# EUROPEAN PATENT APPLICATION

(11) **EP 3 884 924 A1**
(43) Date of publication of application: **29.09.2021**
(21) Application number: 20164997.7
(22) Date of filing: 23.03.2020
(51) Int. Cl.: A61K 8/02, A61Q 1/12

(54) **MULTIPHASE COSMETIC PRODUCT**

(71) Applicant: Omnicos Group S.r.l., 26014 Romanengo (Cremona) (IT)
(72) Inventor: DE PECCATI, Giovanni, 20863 CONCOREZZO (Monza Brianza) (IT); GRANATA, Cristian, 26013 CREMA (Cremona) (IT); CICCHETTI, Domenico, 26010 CAMISANO (Cremona) (IT)
(74) Representative: Lunati & Mazzoni S.r.L.

(57) **Abstract**

A cosmetic device (1) is provided, defining a resting surface (10) and comprising at least one first cosmetic product (2) and one second cosmetic product (3), wherein the second cosmetic product (3) is interposed between at least part of said first cosmetic product (2) and said resting surface (10) and wherein at least part of the second cosmetic product (3) autonomously penetrates at least part of the first cosmetic product (2) with a penetration rate proportional to time.

## Description

This invention relates to a multiphase cosmetic device of the type specified in the preamble of the first claim.

In particular, this invention relates to a multiphase, at least biphasic, cosmetic device, i.e. one that incorporates a plurality of cosmetic products within it.

Similar devices are described in patent applications WO 01/91605 A1, FR 2 985 422 A1, US 2007/059263 A1, WO 2006/125598 A1, and EP 2 220 959 A1.

As is well known, many cosmetics are available in the current state of the art, whether with or without packaging, and which have different consistencies and features.

For example, the cosmetic product may be a mascara, blusher, lip gloss, lipstick, eyeliner, kajal, eye shadow, gloss, foundation, concealer, serum, cream, skincare product, eye contour, or skin care.

Each cosmetic can be liquid, powder, solid, creamy, or colloidal. In addition, cosmetic products can be multiphase, e.g. biphasic, i.e. include a plurality of phases of different materials within them.

An example of this type of cosmetic is the cosmetic that Intercos™ markets under the name Prisma Shine™. Prisma Shine™ is basically a powder that becomes soft to the touch thanks to the gel content of the cosmetic product. Therefore, Prisma Shine™ includes a first powder phase and a second gel phase in which the gel conveys the pigmented powder and enables its distribution over skin ensuring a higher gloss than normal powders.

The described prior art comprises some significant drawbacks.

In particular, multi-layered or biphasic cosmetics basically perform functions that are exclusively the sum of the features of the two products that are used simultaneously. For example, the Prisma Shine™ product only enables the use of soft powder if it is immersed in the gel.

On the other hand, in the case of non-biphasic products, such as common compacted and uncompressed powders, the main drawback is that the cosmetic product has a dry texture that can create discomfort on the skin or, in any case, can lead to skin absorption problems.

In this context, the technical task underlying this invention is to devise a multiphase cosmetic device capable of substantially overcoming at least some of the above-mentioned drawbacks.

In the context of said technical task, an important purpose of the invention is to obtain a multiphase cosmetic device that is capable, if it comprises a powder product, of reducing the dryness of at least one of the cosmetic products, or phases, contained therein.

Another important purpose of the invention is to provide a multiphase cosmetic device to perform the synergistic effect of the phases without all the phases' needing to be used, for example deposited, on the skin at the same time.

The technical task and the specified purposes are achieved by means of a multiphase cosmetic device as claimed in the appended claim 1.

Preferred embodiments are highlighted in the dependent claims.

The characteristics and benefits of the invention will be clarified in the following detailed description of some preferred embodiments of the invention, with reference to the accompanying drawings, wherein:
**Fig. 1** shows a perspective and cross-section view of a multiphase cosmetic device according to the invention in a first embodiment;
**Fig. 2** illustrates a cross-section view of a multiphase cosmetic device according to the invention in a second embodiment; and
**Fig. 3** is a cross-section view of a multiphase cosmetic device according to the invention in a third embodiment.

In this document, when measurements, values, shapes, and geometric references (such as perpendicularity and parallelism) are associated with words like "approximately" or other similar terms, such as "almost" or "substantially", they shall be understood as except for errors of measurement or imprecisions due to errors of production and/or manufacturing and, above all, except for a slight divergence from the value, measurement, shape, or geometric reference with which it is associated. For example, if associated with a value, such terms preferably indicate a divergence of no more than 10% of the value itself.

Furthermore, when used, terms, such as "first", "second", "higher", "lower", "main", and "secondary" do not necessarily identify an order, relationship priority, or relative position, but they can simply be used to distinguish different components more clearly from one another.

Unless otherwise stated, the measurements and data reported in this text shall be considered as performed in International Standard Atmosphere ICAO (ISO 2533:1975).

With reference to the figures, the reference number 1 indicates, as a whole, the multiphase cosmetic device according to the invention.

The device 1 preferably defines its own shape. Therefore, it preferably defines a resting surface **10.**

The resting surface 10 is basically the surface with which the device 1 interfaces with a possible packaging support portion, or a surface designed to enable the device 1 to rest on a plane. Therefore, for example, the resting surface 10 can be basically flat.

The device 1 is preferably a biphasic cosmetic device, i.e. including two phases. However, the device 1 could also be part of a multiphase cosmetic device with more than two phases, or include at least more than two phases itself. The term "phases" refers to the different layers or portions of the device 1 including different cosmetic products.

In fact, the device 1 preferably comprises a first cosmetic product **2** and a second product **3,** which is preferably cosmetic. The term "cosmetic product" generally refers to a product that is mainly used for cosmetic purposes, but could also be a pharmaceutical product, for example the second product 3, or something else.

The first cosmetic product 2 may be of various types.

The first cosmetic product 2 preferably includes cosmetic powder. In particular, this cosmetic powder may be powder formed or processed in various ways. For example, it may be powder that is compact, or extruded, or even injected, or cooked. The second cosmetic product 3 is preferably interposed between at least part of the first cosmetic product 2 and the resting surface 10.

Basically, the first cosmetic product 2 and the second cosmetic product 3 preferably define an at least partially sandwich configuration in relation to the resting surface 10.

However, there are many configurations that can be produced within the context of this inventive concept.

In a first embodiment, the first cosmetic 2 basically forms a containment shell **20.** The containment shell 20 is preferably an open casing in one section, e.g. coinciding with or facing the resting surface 10 in such a way as to at least partially form the resting surface 10. Preferably, the containment shell 20 basically delimits the resting surface 10.

In addition, the shell 20 defines at least one cavity **21.**

Of course, the containment shell 20 could also include a plurality of cavities 21 in such a way as to form different portions of the device 1 wherein the phases defined by the products 2, 3 are different to each other.

The cavity 21 is preferably trapped between the containment shell 20 and the resting surface 10. Therefore, when the device 1 is in use, the cavity is preferably hidden by the containment shell 20 and the device 1 is only accessible via the containment shell 20.

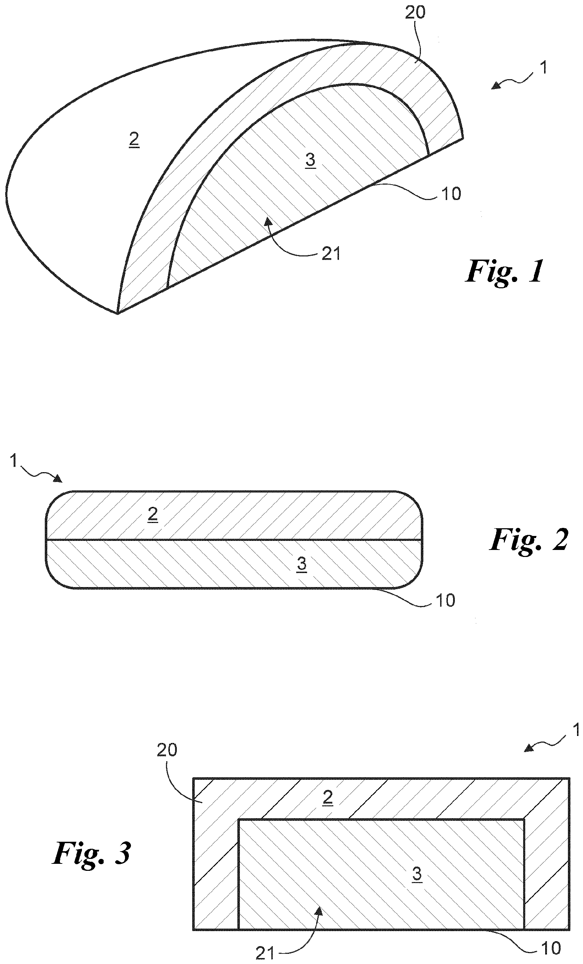

The shapes may be of various types and in different sizes. For example, in the first embodiment, the containment shell 20 is a spherical cap 20 and the cavity 21 extends within a portion of the spherical cap (Fig. 1).

In a second embodiment (Fig. 2), in contrast, the first cosmetic product 2 and the second cosmetic product 3 form two layers, which are basically flat and overlapping each other. Therefore, when the device 1 is in use, the second cosmetic product is also accessible from the outside.

In a third embodiment, similar to the first embodiment, the containment shell 20 is basically square, e.g. in the form of a parallelepiped and covers one layer of the second cosmetic product 3.

As already mentioned, the device 1 may form resting surfaces 100 and, thus, base sections of any shape, for example circumferential, quadrangular, or triangular or other.

In any case, and advantageously, the second cosmetic product 3 autonomously penetrates at least part of the first cosmetic product 2. The term "autonomously" refers to either the way in which the second cosmetic product 3 can penetrate the first cosmetic product 2 spontaneously, e.g. by spontaneous reaction, or in which the second cosmetic product 3 can penetrate the first cosmetic product 2 through externally induced reactions in which, however, penetration is achieved exclusively as a result of the behaviour of the second cosmetic product 3.

For example, penetration created by mechanical pressure on the resting surface 10, so as to crush the second cosmetic product 3 on the first cosmetic product 2, is not autonomous penetration.

In contrast, penetration due to a change of state of the second cosmetic product 3, in which the change of state is achieved by heating the environment outside the device 1, is considered autonomous.

Preferably, the second cosmetic product 3 autonomously penetrates the first cosmetic product 2 with a penetration rate proportional to time.

The term "penetration rate" refers to the penetration depth reached inside the first cosmetic product 2, e.g. inside the containment shell 20.

The penetration rate is preferably, but not necessarily, lower than 50 µm/h. More suitably, the penetration rate can range between 1 µm/h and 50 µm/h. Even more suitably, the penetration rate can be about 5 µm/h.

In other words, once the device 1 has been produced, the second cosmetic product 3 continues to penetrate the first cosmetic product 2 over time in such a way as to deposit at least part of its composition, and its related properties, into the first cosmetic product 2.

In order to achieve this characteristic, the second cosmetic product 3 preferably comprises, at least in part, volatile material.

As is well known, volatility is a chemical-physical property that represents the tendency of a solid or of a liquid to sublimate or evaporate respectively. In general, substances that, under certain pressure and temperature conditions, have high vapour pressure are considered to be volatile. In other words, the system tends to evolve spontaneously towards the aeriform state, reaching the maximum level of stability relative to the conditions in question.

Considering the device 1 including the first cosmetic product 2 and the second cosmetic product 3, at given temperatures and pressures, for example, the relative volatility of the first cosmetic product 2 compared to the second cosmetic product 3 is the ratio between their vapour pressures measured at the set temperature and pressure values.

In particular, the volatile material preferably has a coefficient of volatility, in percentage, equal to or greater than 1%. More suitably, the volatile material has a coefficient of volatility ranging between 1% and 10%.

In general, the second cosmetic product 3 preferably at least partially includes a primer. This primer preferably has the volatility characteristics described above.

A volatile material of this type is, for example, silicone oil or a solvent. Silicone oil, or a solvent, is light and volatile enough to spontaneously penetrate the first cosmetic product 2.

For example, the device 1 may therefore include silicone oil as the second cosmetic product 3 and another product as the first cosmetic product 2.

As an alternative to the above, the second cosmetic product 3 may penetrate the first cosmetic product including by capillarity alone.

The functioning of the device 1 described above in structural terms, is as follows. Once the device 1 has been produced, the second cosmetic product 3 penetrates the first cosmetic product 2 over time in such a way as to deposit, at least partially, inside the first cosmetic product 2. Thus, the second cosmetic product 3 transfers and transmits part of its properties to the first cosmetic product 2. Preferably, the transfer mainly or exclusively involves the active ingredients included in the second cosmetic product 3.

For example, the device 1 may enable the transfer of emollient active ingredients from the second cosmetic product 3 to the first cosmetic product 2.

The invention comprises, in addition, a new method for manufacturing a multiphase cosmetic 1.

The method preferably comprises at least one deep-drawing and one pouring step. In the deep-drawing step, preferably, a wafer including the first cosmetic product 2 is deep-drawn in such a way as to form the containment shell 20 and to define at least one cavity 21.

The wafer of the first cosmetic product 2 may, in turn, be previously formed through an extrusion step.

During the extrusion step, preferably, the cosmetic powder is extruded in such a way as to form the wafer with the first cosmetic product 2. In this extrusion step, preferably, the first cosmetic product 2 is then pre-formed in such a way as to be easily deep-drawn in the following step.

Preferably, the containment shell 20 basically maintains the thickness of the wafer. The wafer is preferably 0.5 mm thick, or thicker. More suitably, the wafer is between 0.5 mm and 20 mm thick. Even more suitably, the wafer is about 10 mm thick. Similarly, following the deep-drawing step, the cavity 21 is 0.5 mm deep, or deeper. More suitably, the cavity 21 is between 0.5 mm and 40 mm deep. Even more suitably, the cavity 21 is about 20mm deep.

In the pouring step, in conclusion, the second cosmetic product 3 is poured into the cavity 21. It is poured into the cavity 21 so that said second cosmetic product 3 is, at least partly, in contact with the first cosmetic product 2 in such a way as to penetrate at least part of the first cosmetic product 2 with a penetration rate proportional to time.

The penetration rate, or gradient, assumes values as described, for example, above. The multiphase cosmetic device 1 according to the invention achieves important advantages.

In fact, the device 1 is able to transfer part of the properties of the second cosmetic product 3 to the first cosmetic product 2 by means of the volatilized active ingredients. Therefore, if the first cosmetic product 2 is extruded powder, the second cosmetic product 3 is able to reduce the dryness of the powder without flooding the powder itself.

A further advantage of the cosmetic product 1 is that it performs the synergistic effect of the phases, i.e. of the cosmetic products 2 and 3, without all phases' needing to be used, e.g. deposited, on the skin at the same time. The interaction between the first cosmetic product 2 and the second cosmetic product 3 only occurs by means of the volatilized active ingredients.

In addition, over time, the volatility of the second cosmetic product 3 ensures that the properties are transmitted continuously over a predetermined time period.

In conclusion, the implementation of the device 1 is simple and economical. Variations may be made to the invention that fall within the scope of the inventive concept defined in the claims.

For example, the first cosmetic product 2 and the second cosmetic product 3 may be directly in contact or separated by means of an interface membrane.

For example, this membrane may be placed between the first cosmetic product 2 and the second cosmetic product 3 and may be permeable or breathable.

In fact, the interface membrane may, preferably, be designed to enable the passage of at least part of the second cosmetic product 3. Examples of this type may include common non-woven fabric sheets, for example those used in medical, SMS or other applications.

In this context, all details can be replaced by equivalent elements, and the materials, shapes, and dimensions may be any materials, shapes, and dimensions.

## Claims

1. A multiphase cosmetic device (1) defining a resting surface (10) and comprising at least one first cosmetic product (2) and one second cosmetic product (3),
- said second cosmetic product (3) being interposed between at least part of said first cosmetic product (2) and said resting surface (10),
and said device (1) being **characterised in that**
- at least part of said second cosmetic product (3) autonomously penetrates at least part of said first cosmetic product (2) with a penetration rate proportional to time and less than 50 µm/h.

2. The device (1) according to claim 1, wherein said penetration rate proportional to time ranges between 1 µm/h and 50 µm/h.

3. The device (1) according to claim 1, wherein said second cosmetic product (3) comprises, at least in part, a volatile material.

4. The device (1) according to claim 1, wherein said volatile material has a coefficient of volatility, in percentage, equal to or greater than 1%.

5. The device (1) according to claim 1, wherein said volatile material is silicone oil.

6. The device (1) according to claim 1, wherein said second cosmetic product (3) penetrates said first cosmetic product (2) by capillarity.

7. The device (1) according to claim 1, wherein said first cosmetic product (2) includes an extruded cosmetic powder.

8. A method for manufacturing a multiphase cosmetic device (1) **characterised in that** it comprises:
- deep-drawing a wafer including a first cosmetic product (2) so as to form a containment shell (20) defining at least one cavity (21),
- pouring a second cosmetic product (3) into said cavity (21) so that said second cosmetic product (3) is at least partly in contact with said first cosmetic product (2) so as to penetrate at least part of said first cosmetic product (2) with a penetration rate proportional to time and less than 50 µm/h.

9. The method according to claim 8, comprising
- extruding a cosmetic powder so as to form said wafer of said first cosmetic product (2).

10. The method according to at least one of claims 8-9, wherein said wafer has a thickness equal to or greater than 0.5 mm.

11. The method according to at least one of claims 8-10, wherein said containment shell (20) is a spherical cap, and said cavity (21) extends into a portion of said spherical cap.

12. The device (1) according to at least one of claims 8-11, wherein said penetration rate proportional to time ranges between 1 µm/h and 50 µm/h.
